# EUROPEAN PATENT APPLICATION

(11) **EP 4 714 374 A1**
(43) Date of publication of application: **25.03.2026**
(21) Application number: 25197985.2
(22) Date of filing: 25.08.2025
(51) Int. Cl.: A61B 17/34, A61B 34/20, A61B 90/30, A61B 90/00

(54) **A SURGICAL TUBE FOR ENHANCED VISUALIZATION IN MINIMALLY INVASIVE SURGERY AND A SURGICAL SYSTEM**

(30) Priority: 23.09.2024 US 202463697920 P; 26.09.2024 US 202418897060
(71) Applicant: Robotron Surgical IP Holding LLC, Miami, FL 33133 (US)
(72) Inventor: SIEMIONOW, Krzysztof, Chicago, IL 60610 (US)
(74) Representative: Kancelaria Eupatent.pl Sp. z o.o.

(57) **Abstract**

A surgical tube (10) comprising: a body (11); and a vision module (20) combined with the body (11), the vision module (20) comprising: a camera (22) mounted at an inner part of a leading end (12) of the surgical tube (10), the camera (22) having a field of view covering a vicinity of an inner volume of the surgical tube (10) at the leading end (12); and a lighting (23) directed towards the field of view.

## Description

### FIELD OF THE INVENTION

The present invention relates to the field of surgery, specifically to minimally invasive procedures including ENT (Ear, Nose, and Throat), neurosurgery, spine surgery, interventional radiology, and pain management surgery. The invention integrates advanced imaging and optionally real-time motion tracking technologies, into surgical tubes to enhance the precision and safety of minimally invasive surgeries by enabling the surgeon a precise real-time view of the surgical field and surgical instrument operating within the field.

### BACKGROUND

Minimally invasive spine surgery has become a widely accepted approach to reduce tissue damage and promote faster recovery. One technique used in this field is the sequential muscle dilation tube approach, which allows for access to the spine with minimal disruption to surrounding muscles. However, visualization of the instrument tip and surrounding anatomy can be limited due to the instrument handles or other obstructions. Similar problems exist in other surgical procedures.

### SUMMARY OF THE INVENTION

The present invention addresses the limitations of current minimally invasive surgery techniques by integrating a small digital camera and lighting at the inner leading end of a surgical tube. This novel design provides a clear and unobstructed view of the surgical field, including the view of the surgical instrument and surrounding anatomy, enhancing the surgeon's ability to navigate and operate within the surgical field. Optionally, other elements are integrated as well, such as an electromagnetic or optical motion tracking sensor.

The body of the surgical tube forms its main structural component, extending from the trailing end to the leading end. It can be made from various materials such as stainless steel, titanium, high-strength polymers, carbon fiber composites, or biocompatible ceramics to ensure durability and sterility. The body can be designed to integrate seamlessly with the vision module or to accommodate it as an attachment.

The trailing end is the part of the tube that extends above the body when the tube is installed during the surgical procedure. In some embodiments, the proximal end may house components like the controller, battery, and wireless communication module.

The leading end is the operational section of the tube that is inserted inside the body.

The vision module is a critical component that provides high-resolution visual data of the surgical field in the vicinity of the leading end of the surgical tube. It is located at the leading end. The vision module includes several sub-components, each contributing to its functionality.

The housing of the vision module encases the components of the vision module.

The camera captures high-resolution still images or videos of the surgical site in the vicinity of the leading end of the surgical tube, i.e., in the area immediately surrounding the leading end of the surgical tube. This includes the surgical site and any anatomical structures within the vicinity of the tube's leading end, typically within a few millimeters to a few centimeters, depending on the specific surgical application and the configuration of the vision module. The field of view of the camera may be set up to encompass a fragment of the leading end or just the area in close proximity to the leading end (such as 1 mm further from the leading end), depending on the needs of the procedure performed. The camera field of view also captures the distal end of the surgical instrument that is inserted into the surgical tube to perform the surgical procedure. The camera can be a high-resolution digital camera, such as a CCD or CMOS sensor, capable of capturing detailed images or video. The size of the camera can be in the order of 1x1 mm. The camera can be equipped with adjustable focus and field of view settings to cater to different surgical needs. In some embodiments, the camera may include features like optical zoom or image stabilization to enhance visual clarity. Additional features such as infrared imaging or fluorescence imaging can be included for specific surgical applications.

The lighting provides illumination to the surgical field, ensuring that the camera captures clear images. The lighting can comprise a light-emitting diode (LED) or a plurality of LEDs that can be positioned around the camera and may include adjustable brightness settings. The size of the LEDs can be as small as 0,2 x 0,2 mm. The LEDs can emit different color temperatures or wavelengths, such as UV or IR, to enhance visibility of specific tissues or structures.

A separator can be provided between the camera and the lighting to prevent light bleeding into the camera and to avoid interference.

The controller manages the operation of the vision module, including the camera and LEDs. It can be located within the housing or at the proximal end. The controller may act as an interface between the data processing module and the components of the vision module and may perform actions such as image pre-processing and data compression. The controller can also include advanced features such as real-time image enhancement algorithms or automatic exposure control.

The battery provides power to the components of the vision module. It can be integrated into the housing of the vision module or located at the trailing end of the tube. The battery may support wireless charging and should offer sufficient capacity to last through lengthy surgical procedures.

The motion tracking indicator tracks the location of the leading end within the surgical field. It can use technologies like electromagnetic or motion tracking sensors. The motion tracking indicator provides real-time positional and orientational data that can be used to generate additional information at the data processing module. This information can be further combined along with positioning information on the distal end of the surgical instrument inserted into the tube, either by receiving a signal from an electromagnetic sensor installed within the surgical instrument or by processing image data obtained via the vision module to recognize the position and orientation of the distal end of the surgical instrument.

The communication module enables data transmission between the vision module and external devices like a data processing module. It can be a wired module, such as a USB interface, or a wireless module that supports various communication protocols such as Wi-Fi, Bluetooth, or proprietary wireless standards.

The components of the vision module, when not housed together, can be connected via a cable that can be routed along the outer periphery of the body of the tube within a dedicated duct. The cable ensures reliable power and/or data transmission between the components. The dedicated duct can be a channel within the body or outside the body that houses the cable. It protects the cable from damage and ensures that it does not interfere with the surgical procedure. The duct can be designed to be easily accessible for maintenance or replacement of the cable.

The surgical tube can be used in a surgical system in connection with a data processing module responsible for handling and analyzing the data received from the vision module. The data processing module processes visual and tube and/or instrument position and orientation data to provide real-time feedback to the surgeon. It can perform tasks such as image enhancement, data fusion, and real-time analytics.

The data processing module can be a standalone unit, such as a dedicated computer system located in the operating room. It can also be an integrated system, embedded within a surgical robot or other medical equipment. Furthermore, it can be implemented in a cloud-based environment, wherein data is transmitted to a remote server for processing and then sent back to the operating room.

The data processing module communicates with the vision module of the surgical tube via a wired or wireless communication interface.

A data processor is the core computational unit within the data processing module. It executes algorithms for image processing, data analysis, and system control. It can be implemented as standard CPUs or GPUs capable of handling complex computations, or specialized FPGAs or ASICs designed for specific tasks like real-time image processing.

An artificial intelligence (AI) module enhances the capabilities of the data processor by adding advanced data analysis features. It performs tasks such as anatomy recognition, segmentation, and annotation using specific algorithms and methods. For anatomy recognition, the AI module may utilize convolutional neural networks (CNNs) trained on large datasets of medical images. Segmentation can be achieved through techniques such as U-Net architectures or region-based convolutional neural networks (R-CNNs), which delineate anatomical structures from the background. Annotation involves labeling the identified structures using pre-trained models. These methods enable the AI module to provide predictive analytics and decision support, enhancing the surgeon's ability to make informed decisions during the procedure.

Visual feedback to the surgeon is provided via an external display, showing real-time images of the surgical site along with the surgical instrument. It can display high-resolution images and augmented reality overlays to enhance situational awareness. The display may take the form of a standard medical-grade monitor, or a wearable head-up display integrated into surgical goggles.

The surgeon can interact with the system via a user interface, adjusting settings of the vision module. It allows control of the vision module and the data processing module, including camera settings, lighting adjustments, and data overlays. For example, the user interface may take the form of a keyboard, a pointer, touch control, hands-free operation using voice commands, or tactile feedback for precise control. Some user interface elements can be integrated with the body of the surgical tube. The user interface can also include customizable presets for different surgical procedures or user preferences.

External data sources may provide additional data that can be integrated into the system for enhanced functionality, such as preoperative imaging data (e.g., MRI or CT scans), patient records, and other relevant information to the data processing module.

Additional information may be generated based on the position signal from the motion tracking indicator, which allows accurate tracking of the tube position and surgical instrument movement within the surgical field. This additional information can be overlaid on preoperative or intraoperative imaging to always inform the surgeon of their position relative to critical anatomical structures, allowing for increased safety and precision of the surgical procedure, leading to better outcomes. The display may be an augmented reality display, for example a head-worn display that allows to overlay the additional information over the real world image as seen by the surgeon. The AI module can recognize and differentiate between various anatomical structures, such as pedicles, nerves, and ligaments. For example, the system can measure the surface area of these structures, providing the surgeon with additional quantitative data to assist in decision-making.

For example, the motion tracking indicator data can be used to create a "painting" effect, where the surgical instrument's contact points on the anatomy are recorded and matched with preoperative MRI or CT scans. The AI system may correlate the real-time camera data with the motion tracking indicator data and preoperative imaging, enhancing the accuracy of the instrument's placement and navigation. This increases the surgeon's situational awareness.

The integration of visual and positional data allows for a more comprehensive understanding of the surgical field. The data from the camera helps refine the positional data from the motion tracking indicator, and vice versa, leading to improved precision in surgical procedures. As a result, there is potential to reduce human error, such as wrong level (or site) surgery, avoid critical neurovascular structures, and ensure that the area of pathology is correctly identified and treated.

The present invention provides a novel and advanced surgical tube designed to enhance the precision and safety of minimally invasive surgeries. Unlike traditional surgical tubes, this invention integrates a vision module with various other components to offer real-time visual and positional data related to the surgical instrument to the surgeon.

The object of the present invention represents a synergistic integration where the combined elements produce new and enhanced results. The vision module, which includes a high-resolution camera, lighting, and optionally positional tracking, works in synergy with the surgical tube to provide real-time visual data on the surgical site and position and orientation data on the surgical instrument. This integration allows for improved situational awareness, precision, and safety during surgery, which is not achievable by the individual components alone. This functionality can be further enhanced by advanced features such as adjustable focus, optical zoom, image stabilization, and real-time data processing through an AI module. These features collectively enhance the surgeon's ability to visualize and navigate the surgical field, reducing the risk of errors and improving patient outcomes.

The surgical system of the present invention allows for real-time fusion of visual and tube and/or instrument position and orientation data. The vision module's camera and lighting provide high-resolution images, while the tracking system ensures accurate tube and instrument placement. This data is processed and analyzed in real-time by an external data processing module, which can overlay additional information, such as anatomical structures and preoperative imaging, onto the live video feed. This level of integration and real-time data fusion is not found in existing surgical systems. Furthermore, the inclusion of an AI module within the data processing system adds the ability to perform tasks such as anatomy recognition, segmentation, and annotation, providing the surgeon with critical information during the procedure. This capability enhances decision-making and situational awareness, leading to safer and more effective surgeries.

In a specific aspect, the invention relates to a surgical tube comprising: a body; a vision module combined with the body, the vision module comprising: a camera mounted at an inner part of a leading end of the surgical tube, the camera having a field of view covering a vicinity of an inner volume of the surgical tube at the leading end; and a lighting directed towards the field of view.

Preferably, the surgical tube further comprises a motion tracking indicator configured to indicate the position and orientation of the leading end of the surgical tube.

Preferably, the motion tracking indicator is further configured to indicate the position and orientation of a surgical instrument operating in the vicinity of the surgical tube.

Preferably, the vision module is contained in a housing integrated with the body of the surgical tube.

Preferably, the housing of the vision module comprises a controller.

Preferably, the housing of the vision module comprises a battery for supplying power to components of the vision module.

Preferably, the battery is configured for wireless charging.

Preferably, the housing of the vision module comprises a communication interface for transmitting signals to and/or from the components of the vision module.

Preferably, the surgical tube further comprises a controller located at a distance from the housing of the vision module.

Preferably, the surgical tube further comprises a battery located at a distance from the housing of the vision module.

Preferably, the surgical tube further comprises a communication interface located at a distance from the housing of the vision module.

Preferably, the surgical tube has the form of a sequential muscle dilation tube.

In another aspect, the invention relates to a surgical system comprising a surgical tube as described herein and a data processing module comprising a data processor configured to process data received from the camera of the vision module.

Preferably, the data processor comprises an artificial intelligence module for performing at least one of recognizing, segmenting, and annotating parts of the anatomy visible in the data received from the camera of the vision module.

Preferably, the data processor is further configured to generate additional information to be integrated with a video signal from the camera.

Preferably, the additional information is generated based on data from preoperative MRI or CT scans.

In another aspect, the invention relates to a method for providing enhanced visualization of image data of a surgical field, the method comprising: providing a surgical system as described herein; receiving image data from the camera of the vision module; processing the image data by the data processing module to generate a processed image; and displaying the processed image on a display.

These and other features, aspects, and advantages of the invention will become better understood with reference to the following drawings, descriptions, and claims.

### BRIEF DESCRIPTION OF DRAWINGS

Various embodiments are herein described, by way of example only, with reference to the accompanying drawings, wherein:
Fig. 1 shows schematically a longitudinal cross-section of the surgical tube;
Fig. 2 shows schematically a close-up view of another cross-section of a leading part of the surgical tube;
Fig. 3 shows an example view of an image obtained from the vision module;
Fig. 4A shows schematically a first embodiment of coupling the vision module with the body of the surgical tube.
Fig. 4B shows schematically a second embodiment of coupling the vision module with the body of the surgical tube.
Fig. 4C shows schematically a third embodiment of coupling the vision module with the body of the surgical tube.
Fig. 4D shows schematically a fourth embodiment of coupling the vision module with the body of the surgical tube.
Fig. 5 shows a functional diagram of a surgical system;
Fig. 6 shows steps of a method for providing enhanced visualization of image data of a surgical field.

### DETAILED DESCRIPTION OF EMBODIMENTS

The following detailed description is of the best currently contemplated modes of carrying out the invention. The description is not to be taken in a limiting sense but is made merely for the purpose of illustrating the general principles of the invention.

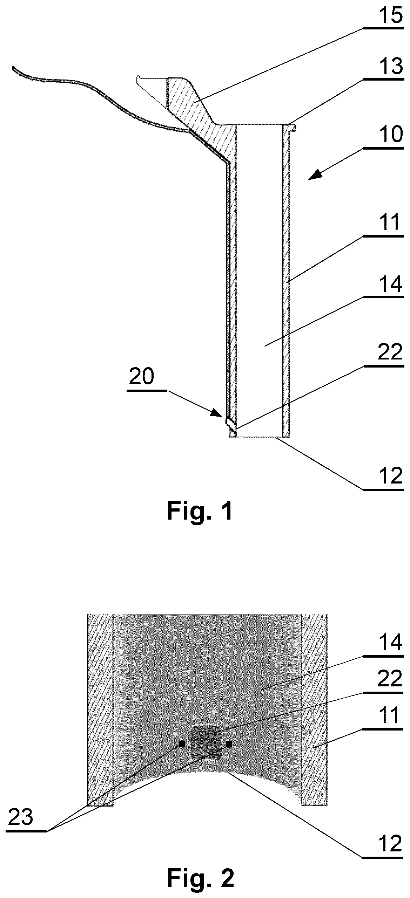

Figs. 1 and 2 show schematically cross-sectional views of an embodiment of the surgical tube 10. The surgical tube 10 has a body 11 with a leading end 12, a trailing end 13 and a through opening 14 extending therebetween. A handle 15 is positioned at the trailing end for manipulating the surgical tube 10. The overall design of the surgical tube can correspond to known designs for surgical tubes adapted for various types of surgical procedures, such as tubes adapted for the sequential muscle dilation tube approach.

A vision module 20 is provided at the leading end 12 of the surgical tube 10, with its field of view covering the inner volume of the through opening 14 and the anatomy visible via the leading end 12. The vision module 20 is configured to capture a real-time view of the surgical site, when the tube is inserted into a patient's body and a surgical instrument is introduced into the tube, such as to allow observation of the distal end of the instrument during the surgical procedure. The vision module 20 contains a camera installed at the inner wall of the body 11 of the surgical tube 10, which generates a video signal that can be displayed on an external display, as will be discussed below.

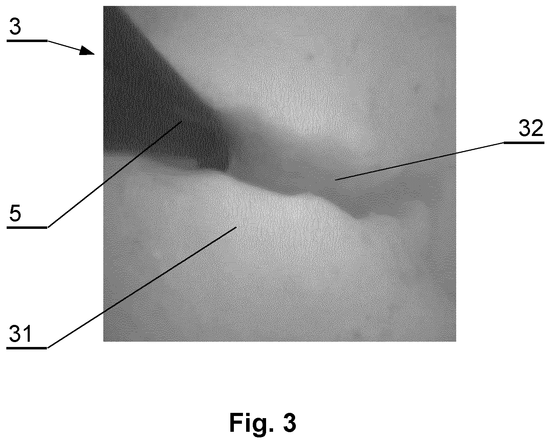

Fig. 3 demonstrates an example of the surgical tube 10 in use. When the surgical tube 10 is inserted into a body, the vision module 20 illuminates the camera's field of view near the leading end 12 of the surgical tube 10. This provides an image of the field of view 3 of the operated area (in this example, related to spine surgery, a bone 31 and a disc space 32 is visible) and any surgical instrument 5 that is inserted into the surgical tube 10, which can be displayed on an external monitor, offering an enlarged view and visibility of the surgical instrument 5 during manipulation in the vicinity of the leading end 12 of the surgical tube 10.

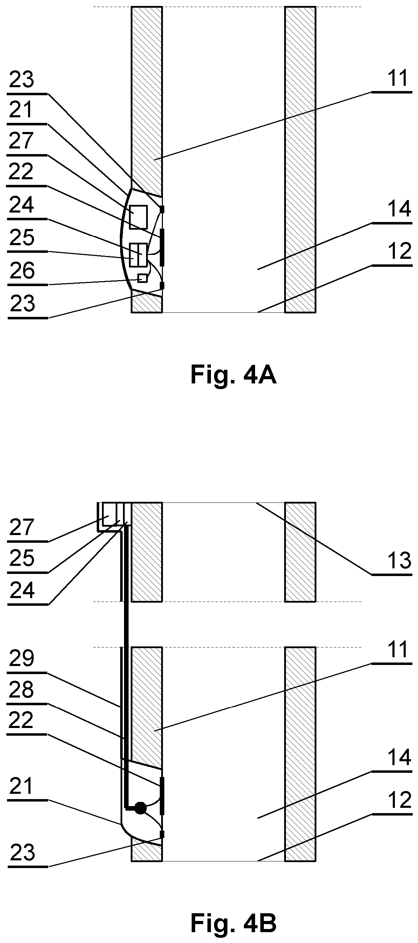

Figs. 4A-4D present various schematic representations of embodiments for coupling the vision module 20 with the body 11 of the surgical tube 10.

In the first embodiment shown in Fig. 4A, the housing 21 of the vision module 20 contains all necessary components for the operation of the vision module 20, including the camera 22, two LEDs 23, a controller 24, a battery 25, a motion tracking indicator 26 (optional), and a wireless communication module 27. The battery 25 can be configured for inductive wireless charging. This design allows the surgical tube 10 to be used like any other surgical tube of this type while providing essential video and position data to an external data processing module. The vision module 20 is embedded in a cutout within the body 11 of the surgical tube 10, such that the camera 22 faces the internal volume of the through opening 14.

In the second embodiment shown in Fig. 4B, the housing of the vision module 20 contains only the camera 22 and a single LED 23. The controller 24, battery 25, and wireless communication module 27 are positioned away from the housing 21 and located at the trailing end 13 of the surgical tube 10. Power and data signals are transmitted between the components via a cable 28 that runs along the outer periphery of the surgical tube 10 in a dedicated duct 29. This design minimizes the size of the housing 21 of the vision module 20.

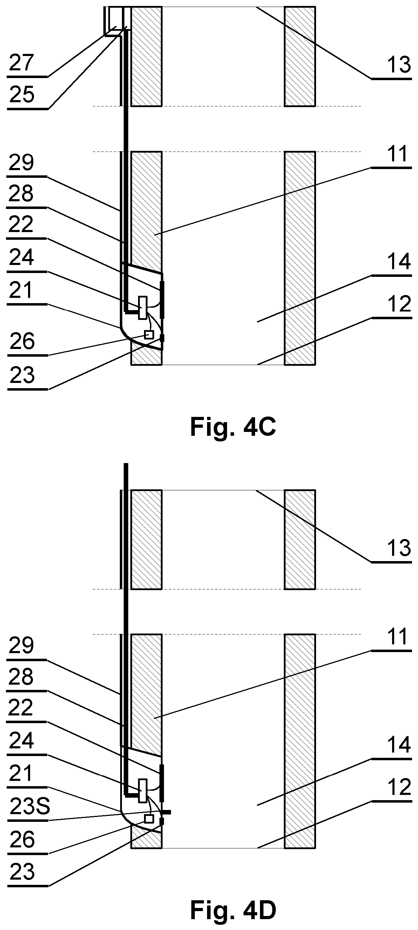

In the third embodiment shown in Fig. 4C, the housing 21 of the vision module 20 contains the camera 22, a single LED 23, the controller 24, and the motion tracking indicator 26. The battery 25 and wireless communication module 27 are positioned away from the housing 21 and located at the trailing end 13 of the surgical tube 10. Power and data signals are transmitted between the components via the cable 28 in the dedicated duct 29.

In the fourth embodiment shown in Fig. 4D, the housing 21 of the vision module 20 contains the camera 22, a single LED 23, the controller 24, and the motion tracking indicator 26. A separator 23S is provided between the camera 22 and the LED 23 to prevent light bleeding into the camera 22 and to avoid interference. Power and data signals are transmitted via the cable 28 that runs in the duct 29 and connects the components within the housing 21 to the external data processing module, which contains the power source.

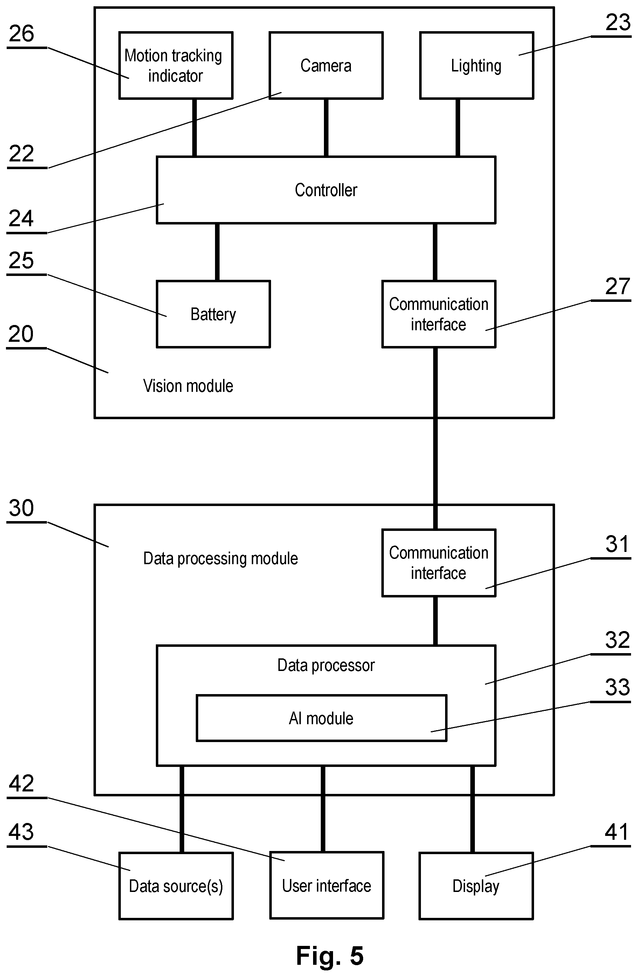

Fig. 5 illustrates a functional diagram of a surgical system comprising a surgical tube 10 equipped with a vision module 20 that communicates with a data processing module 30. The data processing module 30 includes a communication interface 31 for interacting with the communication interface 27 of the vision module 20 and a data processor 32 for processing data received from the vision module 20. If necessary, the data processor 32 can also send power signals to activate the components of the vision module 20 if it lacks a battery 25 or requires additional power. The data processor 32 is responsible for receiving the video signal from the camera 22 and processing it for display on an external display 41, such as a screen or a head-up display worn by the surgeon. The system can be controlled via a user interface 42, such as a keyboard, pointer, or haptic or voice interface, through which the user may control the operation of the vision module 20. This includes adjusting the field of view of the camera 22 e.g., by changing the angle of view or adjusting focus or modifying the intensity of the lighting 23. It may further include dedicated modules, such as an artificial intelligence module 33, for performing additional data processing functions, such as recognizing, segmenting, and annotating visible parts of the anatomy or a surgical instrument, and generating additional information to be integrated with the video signal from the camera 22. The additional information generated by the artificial intelligence module 33 can be based on data obtained from the vision module 20 and data from external sources 43, such as preoperative MRI or CT scans of the patient.

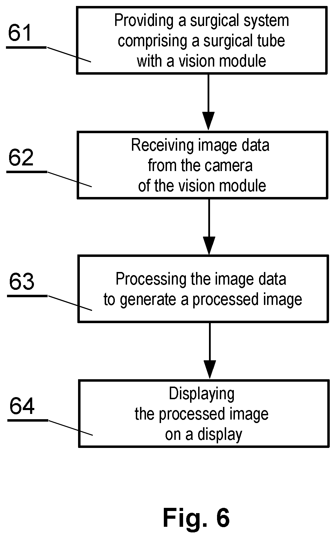

The use of the surgical system as described herein is summarized by the steps of the method shown in Fig. 6, for providing enhanced visualization of image data of a surgical field. In step 61, a surgical system as described herein is provided. Next, in step 62, image data from the camera 22 of the vision module 20 is received by the data processing module 30 and processed in step 63 by the data processing module 30 to generate a processed image 3. The processed image 3 is then displayed in step 64 on a display 41.

While the invention has been described with respect to a limited number of embodiments, it will be appreciated that many variations, modifications, and other applications of the invention may be made, such as combining the features of different embodiments presented herein or simplifying these embodiments by removing some features that are not essential. Therefore, the claimed invention as recited in the claims that follow is not limited to the embodiments described herein.

## Claims

1. A surgical tube (10) comprising:
- a body (11); and
- a vision module (20) combined with the body (11), the vision module (20) comprising:
- a camera (22) mounted at an inner part of a leading end (12) of the surgical tube (10), the camera (22) having a field of view covering a vicinity of an inner volume of the surgical tube (10) at the leading end (12); and
- a lighting (23) directed towards the field of view.

2. The surgical tube (10) of claim 1, further comprising a motion-tracking indicator (26) configured to indicate the position and orientation of the leading end (12) of the surgical tube (10).

3. The surgical tube (10) of claim 2, wherein the motion-tracking indicator (26) is further configured to indicate the position and orientation of a surgical instrument (5) operating in the vicinity of the surgical tube (10).

4. The surgical tube (10) of any of previous claims, wherein the vision module (20) is contained in a housing (21) integrated with the body (11) of the surgical tube (10).

5. The surgical tube (10) of claim 4, wherein the housing (21) of the vision module (20) comprises a controller (24).

6. The surgical tube (10) of any of claims 4 to 5, wherein the housing (21) of the vision module (20) comprises a battery (25) for supplying power to components of the vision module (20).

7. The surgical tube (10) of any of claims 4 to 6, wherein the housing (21) of the vision module (20) comprises a communication interface (27) for transmitting signals to and/or from the components of the vision module (20).

8. The surgical tube (10) of any of claims 4 to 7, further comprising a controller (24) located at a distance from the housing (21) of the vision module (20).

9. The surgical tube (10) of any of claims 4 to 7, further comprising a battery (25) located at a distance from the housing (21) of the vision module (20).

10. The surgical tube (10) of any of previous claims, wherein the surgical tube (10) has the form of a sequential muscle-dilation tube.

11. A surgical system comprising:
- the surgical tube (10) according to any one of claims 1 to 12; and
- a data-processing module (30) comprising a data processor (32) configured to process data received from the camera (22) of the vision module (20).

12. The surgical system of claim 11, wherein the data processor (32) comprises an artificial-intelligence module (33) for performing at least one of recognising, segmenting, and annotating parts of anatomy visible in the data received from the camera (22).

13. The surgical system of claim 11 or 12, wherein the data processor (32) is further configured to generate additional information to be integrated with a video signal from the camera (22).

14. The surgical system of claim 13, wherein the additional information is generated on the basis of data from pre-operative MRI or CT scans (43).

15. A method for providing enhanced visualisation of image data of a surgical field, comprising:
- providing the surgical system according to any of claims 11 to 14;
- receiving image data from the camera (22) of the vision module (20);
- processing the image data by the data-processing module (30) with the data processor (32) to generate a processed image; and
- displaying the processed image on a display (41).
